# EUROPÄISCHE PATENTSCHRIFT

(11) **EP 0 937 443 B1**
(45) Veröffentlichungstag und Bekanntmachung des Hinweises auf die Patenterteilung: **26.05.2004**
(21) Anmeldenummer: 99102555.2
(22) Anmeldetag: 10.02.1999
(51) Int. Cl.: A61F 2/16

(54) **Vorrichtung zum Implantieren einer Intraocularlinse**
Device for introducing an intraocular lens
Dispositif d'introduction pour lentille intra-oculaire

(30) Priorität: 20.02.1998 DE 19807233
(43) Veröffentlichungstag der Anmeldung: 25.08.1999
(73) Patentinhaber: Acritec GmbH, 16548 Glienicke (DE)
(72) Erfinder: Serester, Alexander, 93077 Bad Abbach (DE); Kreiner, Christine F. Dr., 81675 München (DE)
(74) Vertreter: Nöth, Heinz, Dipl.-Phys.

(56) Entgegenhaltungen:
- EP-A- 0 436 232
- WO-A-96/37152
- US-A- 4 699 140
- US-A- 5 284 479

## Beschreibung

Die Erfindung betrifft eine Vorrichtung nach dem Oberbegriff des Patentanspruches 1.

Eine derartige Vorrichtung, welche aus WO 96/37152 A bekannt ist, besitzt in einem Gehäuse einen durch Daumendruck antreibbaren Stößel, bei dessen axialem Vorschub die Intraokularlinse aus dem Gehäuse entfernt und in das Auge implantiert wird. Entgegengesetzt zur Vorschubrichtung des Stößels wirkt eine Feder und/oder ein Dämpfungselement aus elastischem Gummi- oder Kunststoffmaterial, dessen Kraft veränderbar ist.

Ferner ist aus EP 0 477 466 A1 eine Vorrichtung bekannt, bei welcher auf einen Stößel ein Drehantrieb, der als Elektromotor ausgebildet sein kann, über eine Schubstange und ein Getriebe, welches die Drehbewegung in eine Schubbewegung umsetzt, wirkt. Die Intraokularlinse, welche insbesondere als faltbare Intraokularlinse aus gummielastischem Material, beispielsweise Silikon besteht, befindet sich in einem Implantierwerkzeug, das auf die Implantiervorrichtung aufgesetzt werden kann. Die Stößelbewegung in axialer Vorschubrichtung wird auf die Intraokularlinse im Implantierwerkzeug während des Implantierens übertragen.

Aufgabe der Erfindung ist es, eine Vorrichtung der eingangs genannten Art zu schaffen, welche einen einfach gestalteten Antrieb mit steuerbarer Vorschubbewegung aufweist.

Diese Aufgabe wird erfindungsgemäß durch die kennzeichnenden Merkmale des Patentanspruches 1 gelöst.

Bei der Erfindung wirkt auf den Stößel ein Kolben ein, der in Vorschubrichtung von einer Feder, insbesondere Schraubendruckfeder als Kraftspeicher beaufschlagt wird. Entgegengesetzt zur Vorschubrichtung wirkt ein fließfähiges Dämpfungsmedium, insbesondere in Form einer Hydraulikflüssigkeit, die bevorzugt eine ophthalmologisch verträgliche Flüssigkeit sein kann, auf den Kolben ein. Die Wirkung des Dämpfungsmediums kann während des Implantierens von Hand so beeinflusst werden, dass der Kolben und damit der Stößel in Vorschubrichtung von der Feder angetrieben werden. Auf diese Weise erreicht man eine feine Bewegungssteuerung für das Herausbewegen der Intraocularlinse aus dem Implantierwerkzeug, welches mit seiner Spitze in das Auge an den Implantationsort eingesetzt ist.

Die als Kraftspeicher wirkende Feder wirkt auf die eine Kolbenseite und das Dämpfungsmedium wirkt in einem Druckraum bewegungssteuemd aufgrund seiner dosierbaren Dämpfungseinrichtung auf die andere Kolbenseite. Durch die Antriebswirkung der Feder wird im fließfähigen Dämpfungsmedium ein Druck aufgebaut, der durch Öffnen eines Ventils verringert werden kann. Der Öffnungsquerschnitt des Ventils kann dabei von Hand eingestellt werden, so daß die Geschwindigkeit der Vorwärtsbewegung des Kolbens und damit des Stößels beim Ausbringen der Linse aus dem Implantationswerkzeug von Hand steuerbar sind. Auf diese Weise erreicht man, daß die Federvorspannung in einem insbesondere von Hand kontrollierbaren Entspannungshub für das Implantieren der Linse umgesetzt wird.

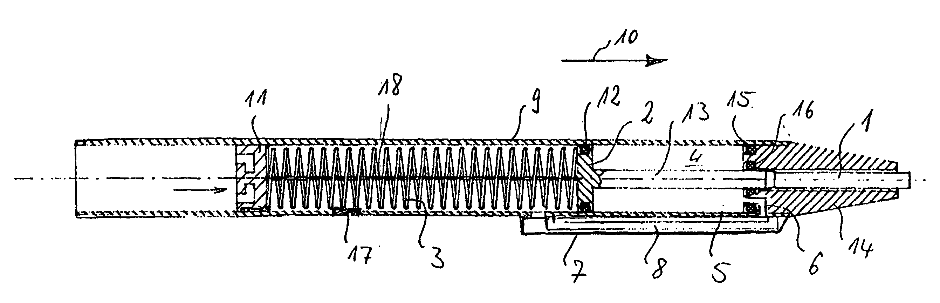

Anhand der Figur wird an einem Ausführungsbeispiel die Erfindung noch näher erläutert.

Die Figur zeigt in schnittbildlicher Darstellung das Ausführungsbeispiel einer Antriebseinrichtung, mit welcher ein Stößel 1 in einem Gehäuse 9 in Vorschubrichtung 10 bewegt werden kann. Im Gehäuse 9 ist als Kraftspeicher eine mechanische Schraubendruckfeder 3 angeordnet. Die Schraubendruckfeder 3 stützt sich über eine Abstützplatte 11 am Gehäuse 9 ab. Die Feder 3 wirkt an einer Seite auf einen verschiebbar im Gehäuse 9 gelagerten Kolben 2. Der Kolben 2 ist druckdicht insbesondere flüssigkeitsdicht beispielsweise mittels eines O-Ringes 12 im Gehäuse 9 geführt. An den Kolben 2 ist eine Schubstange 13 angeformt, die auf den Stößel 1 in axialer Richtung, insbesondere in Vorschubrichtung 10 wirkt.

An der anderen Seite des Kolbens 2 ist in einem Druckraum 5 ein fließfähiges Dämpfungsmedium 4, insbesondere in Form einer Hydraulikflüssigkeit angeordnet. Bei Nichtgebrauch ist ein beispielsweise an einem Verschlußstopfen 14 vorgesehenes Ventil 6 geschlossen. Bei geschlossenem Ventil 6 ist der Druckraum 5 nach außen druckdicht abgeschlossen. Hierzu ist am Verschlußstopfen 14 am Gehäuseende eine Dichtung, beispielsweise in Form eines weiteren O-Ringes 15 vorgesehen. Im Verschlußstopfen 14 wird die Schubstange 13 ebenfalls druckdicht geführt. Dies erfolgt mit Hilfe einer weiteren Dichtung, die ebenfalls als O-Ring 16 ausgebildet sein kann.

Beim dargestellten Ausführungsbeispiel ist ein zum Ventil 6 gehöriger Auslaßkanal vorgesehen. Ferner wird das Ventil 6 ergänzt durch einen Schieber 7, in welchem ebenfalls ein Auslaßkanal 8 mit veränderbarem Querschnitt vorgesehen ist. Der Kanal 8 kann von einem flexiblen Material beispielsweise einem flexiblen Schlauch im Schieber 7 gebildet werden, wobei durch Fingerdruck der Querschnitt des Kanals 8 bzw. des flexiblen Schlauches verringert und erweitert werden kann. Der vom Schlauch gebildete Kanal 8 mündet beispielsweise in den Hohlraum, in welchem die Feder 3 angeordnet ist. Das durch den Kanal 8 aus dem Druckraum 5 verdrängte Druckmedium kann auch in einem anderen Auffangbehälter geleitet werden. Am Schieber 7 kann eine quer zum Kanal 8 bewegliche oder verformbare Platte vorgesehen sein.

Die Feder 3 kann durch Verschieben der Abstützplatte 11 in Vorschubrichtung 10 gespannt werden. Hierzu wird die Abstützplatte 11 eine bestimmte Strecke verschoben und an einer am Gehäuse 9 vorgesehenen Arretierung 17 am Gehäuse fixiert. Hierzu kann die Arretierung 17 am Gehäuse 9 verschiebbar sein, um unterschiedliche Federvorspannungen einstellen zu können. Durch die Verschiebbarkeit der Arretierung werden verschiedene Abstützpositionen der Feder (3) in ihrer axialen Ausdehnung geschaffen. Bei geschlossenem Ventil 6 wirkt die gespannte Feder auf den Kolben 2. Durch das Dämpfungsmedium beispielsweise die Hydraulikflüssigkeit wird jedoch der Kolben 2 an einer Bewegung in Vorschubrichtung gehindert. Durch Öffnen des Ventils 6 beim Verschieben des Schiebers 7 gelangt Dämpfungsmedium insbesondere Hydraulikflüssigkeit durch den Kanal 8 in den Raum, in welchem die Feder 3 angeordnet ist, oder einen anderen Auffangbehälter. Die Feder 3 kann sich entspannen und den Kolben 2 in Vorschubrichtung 10 antreiben, wobei Dämpfungsmedium 4 durch das geöffnete Ventil und den Kanal 8 verdrängt wird. Die Menge des verdrängten Dämpfungsmediums 4 kann durch Regulierung des Ventilquerschnittes insbesondere des Querschnittes des flexiblen Kanals 8 von Hand beispielsweise durch Daumendruck quer zum Kanal 8 reguliert werden. Auf diese Weise läßt sich die Vorschubgeschwindigkeit des Kolbens 2 und des Stößels 1, auf den der Kolben 2 über die Schubstange 13 wirkt, regulieren.

Auf den Verschlußstopfen 14 kann in bekannter Weise mit Hilfe eines Adapters ein Implantationswerkzeug, in welchem die gefaltete Intraocularlinse angeordnet ist, aufgesetzt werden. Der Stößel 1 wird in das Implantationswerkzeug bei seiner Vorschubbewegung bewegt, wodurch die Intraocularlinse aus dem Implantationswerkzeug herausgeschoben wird.

Zur Begrenzung der Vorschubbewegung kann der Kolben 2 mit der Abstützplatte 11 über einen Faden 18 verbunden sein. Eine Einstellung der maximalen Vorschubbewegung erreicht man durch die Einstellung der axialen Verschiebebewegung der Abstützplatte 11 zur entsprechend eingestellten Arretierung 17 am Gehäuse 9. Die Feder 3 kann auch schon beim Zusammenbau der Vorrichtung gespannt werden, so daß sie für den Implantationsvorgang vorbereitet ist.

Der Faden 18 kann auch zum Zurückziehen des Kolbens 2 verwendet werden. Hierdurch ist es möglich, Hydraulikflüssigkeit 4 in den Druckraum 5 durch Aufziehen einzufüllen. Beim Zurückziehen des Kolbens 2 entsteht im Druckraum 5 ein Unterdruck, so daß durch das offene Ventil 6 Hydraulikflüssigkeit 4 angesaugt werden kann. Die Abstützplatte 11 kann dabei mit Hilfe eines speziellen Werkzeugs, beispielsweise Schlüssels, nach hinten gezogen werden und nach dem Füllen des Druckraumes 5 wieder nach vorne bis zur Arretierung 17 zur Einstellung der gewünschten Federspannung verschoben und eingerastet werden. Das Implantierwerkzeug ist dann wieder in Funktionsbereitschaft.

## Patentansprüche

1. Vorrichtung zum Implantieren einer Intraokularlinse mit einem in einem Gehäuse (9) angeordneten Antrieb für einen Stößel (1), welcher durch den Antrieb axial in einer Vorschubrichtung bewegbar ist, wobei diese Bewegung auf eine am Gehäuseende angeordnete Intraokularlinse während des Implantierens übertragbar ist, und wobei auf den Stößel (1) ein Kolben (2) wirkt, der von einer Feder (3) beaufschlagt ist, und entgegengesetzt zur Vorschubrichtung von einem Dämpfungsmedium (4) beaufschlagt ist, dessen Wirkung bei einem Kolbenhub während des Implantierens steuerbar ist,
**dadurch gekennzeichnet, dass** die Feder (3), welche in Vorschubrichtung einen Kraftspeicher bildet, an der einen Kolbenseite und das Dämpfungsmedium (4), welches fließfähig ausgebildet ist, in einem Druckraum (5) auf der anderen Kolbenseite im Gehäuse (9) vorgesehen sind.

2. Vorrichtung nach Anspruch 1, **dadurch gekennzeichnet, daß** während des Imptantierens eine nachlassende Dämpfungswirkung einstellbar ist.

3. Vorrichtung nach Anspruch 1 oder 2, **dadurch gekennzeichnet, daß** die Dämpfungswirkung von Hand einstellbar ist.

4. Vorrichtung nach einem der Ansprüche 1 bis 3, **dadurch gekennzeichnet, daß** die Feder (3) als mechanische Druckfeder ausgebildet ist.

5. Vorrichtung nach einem der Ansprüche 1 bis 4, **dadurch gekennzeichnet, daß** zur Einstellung der Dämpfungswirkung aus dem Druckraum (5) Dämpfungsmedium (4) entfernbar ist.

6. Vorrichtung nach einem der Ansprüche 1 bis 5, **dadurch gekennzeichnet, daß** der in dem Dämpfungsmedium (4) durch die Wirkung der Feder (3) aufgebaute Druck durch ein von Hand steuerbares Ventil (6) verringerbar ist.

7. Vorrichtung nach einem der Ansprüche 1 bis 6, **dadurch gekennzeichnet, daß** das Dämpfungsmedium (4) eine Hydraulikflüssigkeit ist.

8. Vorrichtung nach einem der Ansprüche 1 bis 7, **dadurch gekennzeichnet, daß** der Ventilquerschnitt (8) von Hand einstellbar ist.

9. Vorrichtung nach einem der Ansprüche 1 bis 8, **dadurch gekennzeichnet, daß** verschiedene Vorspannungen der Feder (3), insbesondere durch unterschiedliche Abstützstellen der Feder am Gehäuse (9) einstellbar sind.

10. Vorrichtung nach einem der Ansprüche 1 bis 9, **dadurch gekennzeichnet, daß** durch Zurückziehen des Kolbens (2) Dämpfungsmedium (4) in den Druckraum (5) einfüllbar ist.

## Claims

1. A device for implanting an intraocular lens comprising a drive arranged in a housing (9) for a pushrod (1) which is movable axially in an advance direction by the drive, wherein that movement can be transmitted during the implantation operation to an intraocular lens arranged at the end of the housing and wherein acting on the pushrod (1) is a piston (2) which is acted upon by a spring (3) and is acted upon in opposite relationship to the advance direction by a damping medium (4), the action of which is controllable in a piston stroke during the implantation operation, **characterised in that** the spring (3) which in the advance direction forms a force storage means is provided at the one side of the piston and the damping medium (4) which is adapted to be capable of flow is provided in a pressure chamber (5) on the other side of the piston in the housing (9).

2. A device according to claim 1 **characterised in that** a diminishing damping action can be set during the implantation operation.

3. A device according to claim 1 or claim 2 **characterised in that** the damping action can be set by hand.

4. A device according to one of claims 1 to 3 **characterised in that** the spring (3) is in the form of a mechanical compression spring.

5. A device according to one of claims 1 to 4 **characterised in that** damping medium can be removed from the pressure chamber (5) for setting the damping action.

6. A device according to one of claims 1 to 5 **characterised in that** the pressure built up in the damping medium (4) by the effect of the spring (3) can be reduced by a valve (6) which is controllable by hand.

7. A device according to one of claims 1 to 6 **characterised in that** the damping medium (4) is a hydraulic fluid.

8. A device according to one of claims 1 to 7 **characterised in that** the valve cross-section (8) can be set by hand.

9. A device according to one of claims 1 to 8 **characterised in that** different biaising effects for the spring (3) can be set in particular by different support locations for the spring on the housing (9).

10. A device according to one of claims 1 to 9 **characterised in that** damping medium can be introduced into the pressure chamber (5) by retraction of the piston (2).

## Revendications

1. Dispositif d'implantation d'une lentille intraoculaire comportant, disposé dans une enveloppe (9), un entraînement pour un coulisseau (1) qui peut être déplacé axialement par l'entraînement dans un sens d'avance, ce mouvement étant transmissible pendant l'implantation à une lentille intraoculaire disposée à l'extrémité de l'enveloppe, et un piston (2), qui est sollicité par un ressort (3) et est sollicité à l'opposé du sens d'avance par un fluide d'amortissement (4) dont l'action peut être commandée lors d'une course du piston pendant l'implantation, agissant sur le coulisseau (1),
**caractérisé en ce que** sont prévus dans l'enveloppe (9), d'un côté du piston, le ressort (3) qui constitue un accumulateur d'énergie dans le sens d'avance et, dans une chambre de pression (5) de l'autre côté du piston, le fluide d'amortissement (4) qui est réalisé avec capacité d'écoulement.

2. Dispositif selon la revendication 1, **caractérisé en ce qu'**une action d'amortissement décroissante est réglable pendant l'implantation.

3. Dispositif selon la revendication 1 ou 2, **caractérisé en ce que** l'action d'amortissement est réglable manuellement.

4. Dispositif selon l'une des revendications 1 à 3, **caractérisé en ce que** le ressort (3) est réalisé en tant que ressort de pression mécanique.

5. Dispositif selon l'une des revendications 1 à 4, **caractérisé en ce que** du fluide d'amortissement (4) peut être retiré de la chambre de pression (5) pour régler l'action d'amortissement.

6. Dispositif selon l'une des revendications 1 à 5, **caractérisé en ce que** la pression établie dans le fluide d'amortissement (4) sous l'action du ressort (3) peut être réduite par une vanne (6) pouvant être commandée manuellement.

7. Dispositif selon l'une des revendications 1 à 6, **caractérisé en ce que** le fluide d'amortissement (4) est un liquide hydraulique.

8. Dispositif selon l'une des revendications 1 à 7, **caractérisé en ce que** la section transversale (8) de la vanne est réglable manuellement.

9. Dispositif selon l'une des revendications 1 à 8, **caractérisé en ce que** diverses précontraintes du ressort (3) sont réglables, en particulier par des points d'appui différents du ressort sur l'enveloppe (9).

10. Dispositif selon l'une des revendications 1 à 9, **caractérisé en ce que** du fluide d'amortissement (4) peut être introduit dans la chambre de pression (5) par recul du piston (2).
